Europäisches Patentamt

(19) European Patent Office  (11) Numéro de publication: **0 160 650**

Office européen des brevets  **B1**

(12)  **FASCICULE DE BREVET EUROPÉEN**

(45) Date de publication du fascicule du brevet:
28.02.90

(21) Numéro de dépôt: 84900284.5

(22) Date de dépôt: 29.12.83

(86) Numéro de dépôt international:
PCT/FR 83/00267

(87) Numéro de publication international:
WO 84/02652 (19.07.84 Gazette 84/17)

(51) Int. Cl. ⁵: **A 61 K 35/80, A 23 L 1/337,
A 61 K 7/48**

(54) NOUVEAU PRODUIT PHYSIOLOGIQUE EXTRAIT D'ALGUES ET DE PLANTES, PROCEDE DE PREPARATION, APPAREILLAGE D'EXTRACTION ET APPLICATIONS.

(30) Priorité: 30.12.82 FR 8222136
30.12.82 FR 8222137
30.11.83 FR 8319142

(43) Date de publication de la demande:
13.11.85 Bulletin 85/46

(45) Mention de la délivrance du brevet:
28.02.90 Bulletin 90/09

(84) Etats contractants désignés:
AT BE CH DE FR GB LI LU NL SE

(56) Documents cité:
EP-A-0 075 522
FR-A-2 196 343
FR-A-2 287 943
FR-M-5 576
GB-A-2 022 459
GB-A-2 091 585

Le dossier contient des Informations techniques
présentées postérieurement au dépôt de la demande
et ne figurant pas dans le présent fascicule.

(73) Titulaire: LABORATOIRES GOEMAR S.A.
Avenue du Général Patton Botte Postale 55
F-35403 Saint-Malo (FR)

(72) Inventeur: HERVE, René, Ange
Les Tertres B.P. 6
F-22130 Plancoet (FR)

(74) Mandataire: Richebourg, Michel Franfois
Cabinet Beau de Loménie 55, rue d'Amsterdam
F-75008 Paris (FR)

LIBERGRAF, STOCKHOLM 1990

## Description

La présente invention concerne un procédé complexe de traitement d'algues et de plantes diverses au cours duquel sont combinées plusieurs étapes précises de traitement, grâce auquel on a pu obtenir un produit d'extraction tout à fait original, ci-après dénommé "filtrat physiologique".

On sait traiter les algues et plantes par "cryobroyage" selon le brevet français HERVE et ROUILLER n°74-35162 déposé le 18 octobre 1974.

Selon ce brevet, on coupe puis congèle les algues et plantes à -20/-50°C, puis on les broie à cette température dans un broyeur à couteaux/broyeur à dents disposés en série. Une description détaillée de l'appareillage est donnée dans ce brevet. On envoie ensuite les algues et plantes (dimension moyenne 0,1 - 0,5 mm) vers un ou plusieurs broyeurs supplémentaires, à la température ambiante, par exemple broyeurs à trois cylindres (on atteint ainsi 100 à 50 μm ou même 10 μm). Les caractéristiques de ces appareils sont également décrites dans ledit brevet.

On obtient ainsi une bouillie verte épaisse de pH voisin de 4,8 qui, grâce au cryobroyage, contient la quasi-totalité des substances utiles et bénéfiques contenues dans les algues et plantes de départ (cf. Tableau I du brevet précité).

Cependant, ce produit n'est pas stérile, et ne peut être utilisé tel quel dans le domaine de l'alimentation par exemple.

Par ailleurs, le document GB-A-2 022 459 décrit un broyage moléculaire d'algues après traitement, dans un premier temps, par un broyeur colloïdal.

Là encore, le produit obtenu par ce procédé n'est pas stérile et ses propriétés en limitent le champ des applications.

Enfin, le document FR-2 196 343 décrit de façon générale l'application de l'ultrafiltration à une solution aqueuse de polysaccharide provenant d'algues marines. Cette ultrafiltration conduit à une concentration, éventuellement des germes bactériens, et ne conduit donc pas à un produit stérile.

La présente invention concerne un produit nouveau, dénommé "filtrat physiologique", obtenu par un procédé complexe et spécifique de traitements d'algues ou de plantes diverses au cours desquels plusieurs étapes de traitement sont combinées.

Plus précisément, la présente invention concerne un produit physiologique stérile résultant du traitement d'algues ou de plantes entières par un procédé du type comprenant une étape de cryobroyage consistant en une congélation des algues préalablement coupées, à -20/-50°C, suivie d'un broyage à cette température, dans un broyeur à couteaux ou à dents, puis d'un écrasement desdites algues broyées, dans un ou plusieurs broyeurs, à la température ambiante, afin d'obtenir une bouillie de dimension de particules de 100 à 50 ou 10 μm, caractérisé en ce qu'il comprend, à la suite de cette première étape de cryobroyage, les étapes successives suivantes:

— broyage moléculaire dans des conditions choisies pour permettre l'éclatement cellulaire et la libération d'une grande quantité de liquide intracellulaire;
— décantation à grande vitesse;
— ultrafiltration, par exemple sur une membrane possédant un pouvoir de coupure inférieur à 20 000, en atmosphère stérile, de façon à récupérer ledit extrait sous forme d'un liquide stérile.

En particulier, ce "filtrat physiologique" est exempt de pavé cellulaire, de cellulose, de composants à longue chaîne moléculaire, et est directement assimilable (il contient micro-éléments, vitamines, micropeptides).

Une des caractéristiques essentielles est de ne pas contenir d'alcools résiduels provenant des étapes d'extraction.

Il est utilisable en oligothérapie équilibrée d'appoint et pour favoriser par effet de synergie l'assimilation d'oligoéléments et de vitamines, à raison de 10 à 50 ml de produit pur par jour et par adulte.

Selon un autre aspect, la présente invention a pour objet un procédé de préparation de ce nouveau produit du type comprenant une étape de cryobroyage d'algues ou de plantes entières consistant en une congélation des algues, préalablement coupées, à -20/-50°C, suivie d'un broyage à cette température, dans un broyeur à couteaux ou à dents, suivie d'un écrasement desdites algues broyées, dans un ou plusieurs broyeurs, à la température ambiante, afin d'obtenir une bouillie de dimension de particules de 100 à 50 ou 10 μm, caractérisé en ce qu'il comprend à la suite de cette première étape de cryobroyage, les étapes successives suivantes:

— broyage moléculaire dans des conditions choisies pour permettre l'éclatement cellulaire et la libération d'une grande quantité de liquide intracellulaire;
— décantation à grande vitesse;
— ultrafiltration, par exemple sur une membrane possédant un pouvoir de coupure inférieur à 20 000, en atmosphère stérile, de façon à récupérer ledit extrait sous forme d'un liquide stérile.

Selon un aspect supplémentaire la présente invention a pour object un appereillage pour la mise en oeuvre de ce procédé, caractérisé en ce qu'il comporte la combinaison de broyeurs réfrigérés à -20/50°C, de broyeurs à température ambiante, d'un broyeur moléculaire, d'un décanteuse à grande vitesse et d'un appareil d'ultrafiltration.

Selon un autre aspect, la présente invention a pour object une utilisation de ce nouveau produit pour la préparation de médicaments, d'aliments ou de boissons, ou de compositions cosmétiques.

Les algues ou plantes fraîches sont coupées puis subissent un cryobroyage tel que décrit ci-dessus.

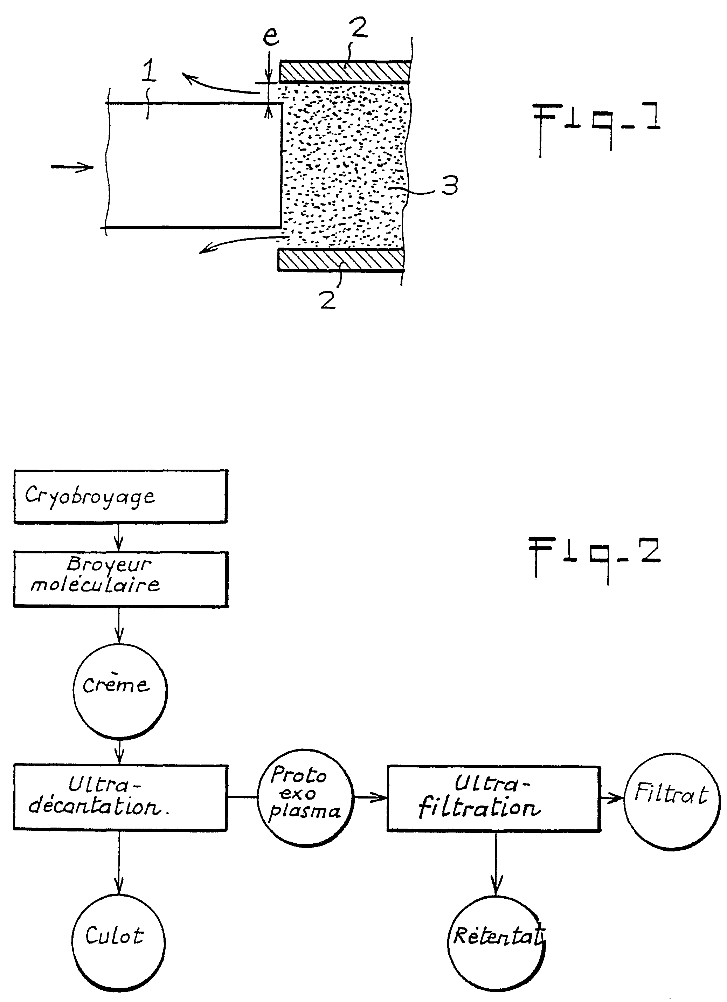

La bouillie obtenue est alors envoyée dans un broyeur moléculaire spécifique tel que représenté sur la figure 1 annexée.

Le broyeur spécifique nécessaire à l'obtention du produit selon l'invention consiste en un ensemble piston (1) clapet (2) comportant entre le piston et le clapet un espace (e) extrêmement faible (de l'ordre de 0,035 mm sous 350 bars et à 400 litres/h).

Le principe est connu, le piston, par son mouvement de va-et-vient, chasse le produit à traiter (3) par l'espace (e) où ce produit est broyé.

On obtient selon ce procédé un produit broyé d'une manière nouvelle, ce qui libère d'autres principes que ceux libérés par les procédés connus, et de manière différente.

Ceci peut peut-être s'expliquer par l'effet de synergie que l'on observe, au niveau du broyage, entre les deux types de broyeurs.

Le broyage moléculaire modifie considérablement le produit, puisqu'il fait passer son temps d'écoulement de, par exemple, 24 s à 59 s (produit beaucoup plus visqueux).

Il est également essentiel de souligner que, pour la première fois, on peut obtenir à la sortie des broyeurs un produit *stérile*, ce qui est d'une importance décisive dans les applications envisagées.

Le procédé d'éclatement reposant sur une baisse brutale de la pression, qui peut devenir négative (effet de cavitation), entraîne, outre l'éclatement des cellules végétatives, l'éclatement des micro-organismes et en particulier des bactéries. On peut ainsi obtenir un produit stérile à condition toutefois que la pollution bactérienne initiale ne soit pas trop élevée.

La combinaison des deux broyeurs permet donc d'obtenir:

– un produit plus actif du fait du plus grand pourcentage de cellules éclatées libérant une plus grande quantité de liquide intracellulaire;
– une meilleure conservation des principes actifs, en particulier des propriétés enzymatiques conservées grâce au maintien d'une basse température au cours de toute la chaîne des opérations;
– des produits présentant une bonne tenue dans le temps grâce à la stérilisation obtenue au moment du broyage (éclatement au cours de la compression-décompression); cette qualité est particulièrement importante dans le cas d'applications cosmétiques.

De plus, la combinaison avec la technique de cryobroyage autorise le traitement des algues ou des plantes *entières*, pour la première fois.

La combinaison d'étapes selon l'invention ne réalise donc pas une extraction mais une "réduction" de la plante entière sous une forme microéclatée.

Enfin, la présence du broyeur "moléculaire" ou "cellulaire" dans la chaîne permet de mettre en oeuvre une ultra-filtration impossible à effectuer sans ce broyeur. Entre le broyage cellulaire et l'ultra-filtration, on effectue une ultra-décantation, elle aussi indispensable.

Le procédé selon l'invention consiste par conséquent en la combinaison des étapes suivantes:

– "cryobroyage",
– passage au broyeur moléculaire,
– ultra-décantation,
– ultra-filtration.

Pour certains produits, bien déterminés, on pourrait envisager de renoncer au broyeur moléculaire mais pas dans le cas général.

L'étape 3) consiste en une turbodécantation avec pipettage du produit au centre du bol.

L'étape 4) d'ultra-filtration est effectuée sur des filtres de carbone comportant une couche d'oxyde de zirconium, membrane M4 [possédant un pouvoir de coupure inférieur à 20 000 (séparant les poids moléculaires) > 20 000)]. Cette étape d'ultra-filtration participe à l'acquisition du caractère stérile.

Après l'étape 4), on conditionne en salle stérile ("salle blanche" ou "clean room") pour préserver la stérilité du produit qui est l'un des résultats essentiels de l'invention.

Cette chaîne d'opérations ainsi que les produits obtenus sont représentés sur la figure 2 annexée.

Dans le cas des algues, le "culot de centrifugation" contient principalement des débris cellulaires et des alginates. Il peut être utilisé pour l'activation de composts et pour le traitement de certains effluents.

Le "rétentat" comprend des molécules de poids moléculaire supérieur à 20 000, en particulier des polysaccharides.

Il peut être utilisé pour la production de produit, à usage externe mettant à profit les propriétés heparin-like des polysaccharides sulfurylés.

Le filtrat contient pratiquement tous les oligo-éléments, 80 % des polysaccharides, les phytohormones et des vitamines et, d'une manière générale, toutes les molécules de poids inférieur à 20 000.

Il peut être utilisé pour la fabrication de teinture - mère homéopathique, de produits cosmétiques, de produits phytothérapeutiques et de boissons.

Les exemples suivants illustrent l'invention sans toutefois en limiter la portée.

**Exemple 1: Filtrat d'algues** (Laminaria).

Le filtrat physiologique "GYFL" est un liquide protocellulaire d'algues fraîches sélectionnées (notamment Laminaria), et hautement purifié par un procédé physique sous froid (cryobroyage, broyage moléculaire, turbodécantation, et ultra-filtration sélective).

– Cryobroyage et broyage moléculaire:
Les algues fraîches sont broyées sous froid (-40°C) permettant d'obtenir une crème d'algues dont la finesse est inférieure à 20 μ.

– Turbodécantation:
La crème d'algues est décantée à grande vitesse permettant d'éliminer la cellulose et les alginates insolubles.

– Ultra-filtration sélective:
Une filtration ultra-fine permet d'obtenir un liquide liquide sans impuretés et dont les composants ont un poids moléculaire inférieur à 20 000.

**Propriétés:**

– Exempt de pavé cellulaire, cellulose, composants à longue chaîne moléculaire, le filtrat physiologique d'algues GYFL est directement assimilable (micro-éléments, vitamines, micropeptides).
L'assimilation est favorisée par une répartition modifiée de poids moléculaire: % de molécules de PM faible est supérieur au % de molécules à PM élevé.

– Le procédé de fabrication permet d'obtenir un produit de haute qualité bactériologique.
– Sans conservateur, sans colorant, sans alcool.

**Utilisations En Diététique:**

– Oligothérapie équilibrée d'appoint.
– Vecteur d'assimilation d'oligo-éléments et de vitamines.
– A utiliser dilué à la dose de 10 à 50 ml de produit pur par jour.

On trouvera ci-dessous les résultats d'analyses effectuées sur ce produit.

**Matières Sèches — Protéines — Lipides — Glucides — Cendres**

Méthodes utilisées pour le dosage des protéines, glucides, lipides et des cendres du filtrat "GYFL".

1°) *Dosage des protéines*
La teneur en protéines est évaluée à partir du taux d'azote total que multiplie 6,25.

2°) *Dosage des lipides*
La technique utilisée est celle décrite par BLIGH AND DYER (1959).
A l'échantillon pesé (contenant 80 g d'eau) est ajouté 300 ml d'une solution chloroforme-méthanol (1 - 2) sous bonne agitation. L'agitation est maintenue 10 mn.
Le résidu insoluble est éliminé par filtration sur papier WHATMAN 1 PS.
A la solution limpide ainsi obtenue sont ajoutés successivement 100 ml de chloroforme et 100 ml de méthanol.
On agite après chaque addition.
Le mélange biphasique obtenu est transféré dans une ampoule à décanter. On laisse reposer une nuit.
La phase chloroformique lourde est recueillie puis évaporée à sec.
Le résidu lipidique est repris par une petite quantité de chlorure de méthylène.
Cette solution est évaporée dans un tube taré et pesé. E.G.BLIGH and W.J. DYER (1959)
A rapid method of Total Lipid Extraction and Purification Canada I. of Biochem and Physial 37 (8), 911.

3°) *Dosage des glucides*
La méthode utilisée est celle de DUBOIS et Col. (1956).
Le mode opératoire utilisé est décrit ci-après. Les résultats sont exprimés en équivalent glucose.
*"Dosage des sucres par la méthode DUBOIS"* – Objet et domaine d'application
Il s'agit d'une méthode colorimétrique, simple, rapide et sensible, applicable aux sucres réducteurs (hexoses, pentoses, disaccharides, polysaccharides), à leurs dérivés méthylés et aux acides uroniques. Il faut signaler que les glucosamines et galactosamines ne sont pas pris en compte.

*– Réactifs*

- PHENOL rectapur, PROLABO (préparer une solution 5 %)
- Acide sulfurique NORMAPUR PROLABO (98,08 %, d = 1,83)
- Solution standard de glucose ou de galactose 10 mg pour 50 ml)

*– Matériel*

- un spectophotomètre visible (lampe au tungstène) (cuves en verre OS)
  L'analyse a été réalisée sur un appareil ACTA III de BECKMAN
- un VORTEX
- une série de tubes de 15 ou 20 ml
- une série de micropipettes de type EPPENDORF.

*– Mode opératoire*

- courbe étalon: prélever entre 20 et 200 µg de standard qu'on ajuste à 2 ml d'$H_2O$.
- Ne pas oublier de prévoir un blanc.
- Ajouter 1 ml de phénol à 5 %
- Bien agiter au vortex
- Ajouter 5 ml d'$H_2SO_4$ concentré, agiter et laisser se développer la coloration.

*– Lire l'absorption à 485 nm au bout de 30 minutes*
M.DUBOIS, K.A. GILLES, J.K. HAMILTON, P.A. REBERS AND F. SMITH (1956) "Colorimetric method for determination of sugars and related substances". Analytical Chemistry 28 (3), 350.

4°) *Dosage des cendres*
La quantité de matière minérale est évaluée par le taux de cendres restant après combustion.
10 à 20 g d'échantillon sont étalés au fond d'une boîte de Pétri tarée.
L'échantillon est d'abord séché à 80°C à l'étuve puis transféré dans un four.
La montée en température se fait de 20°C à 550°C en 24 heures. On maintient à cette température pendant 48 heures.
On pèse après refroidissement complet dans un dessicateur.
Du fait de la faible quantité de matière première disponible, le dosage des lipides de GYFL a été effectué sur 1 ml. De ce fait, la valeur donnée est approximative. Une première mesure a donné 0,03 %, une deuxième 0,04 %.
Les résultats sont rassemblés dans les Tableaux I à III ci-après.
On a également déterminé l'indice d'irritation primaire d'un filtrat physiologique GYFL selon la méthode décrite au Journal Officiel du 21 février 1982.
L'essai est réalisé avec six lapins mâles Albinos, de souche définie de 2,5 kg environ, ayant séjourné une semaine au minimum dans l'animalerie en cage individuelle afin d'être parfaitement acclimatés. Lors de l'essai, les lapins sont maintenus dans des boîtes à contention. La veille de l'application du produit à tester, ils sont tondus sur le dos et les flancs à la tondeuse électrique munie d'un peigne de hauteur de coupe de 0,05 mm pour dégager une surface d'environ 14 x 14 cm. Avant de commencer le test nous nous assurons que les animaux présentent une peau saine et glabre.
A jour J.O, sur le flanc droit de chaque lapin, nous effectuons, à l'aide d'un vaccinostyle stérile, trois scarifications parallèles sur une longueur d'environ 2,5 cm espacées de 0,5 cm environ, sans atteindre le derme, c'est-à-dire sans saignement.
0,5 ml de filtrat physiologique GYFL sont déposés à l'aide d'une seringue stérile de 1 ml au dixième sur deux pièces de gaze hydrophile Codex à 8 épaisseurs, d'une surface de 6,2 $cm^2$, dont une des surfaces est recouverte d'une pellicule adhésive hypoallergénique et imperméable, et appliquées immédiatement sur la peau, maintenues en contact avec la peau sur chacune des deux zones par une bande de fixation microporeuse adhésive hypoallergénique de largeur 50 mm. On place ensuite une compresse de protection en gaze hydrophile Codex de dimensions appropriées; l'ensemble est maintenu par une bande tissée.
Vingt quatre heures après l'application du produit, on évalue l'irritation primaire cutanée. Elle est évaluée 30 minutes après enlèvement du pansement et de nouveau quarante huit heures plus tard, c'est-à-dire soixante douze heures après l'application du produit.
Les observations sont faites sur les deux zones, scarifiée et non scarifiée, selon l'échelle numérique suivante:

*– Erythème et formation d'escarres:*

Pas d'érythème ................................................................................................ 0
Léger érythème ................................................................................................ 1

Erythème bien visible.................................................................................2
Erythème important....................................................................................3
Erythème grave..........................................................................................4
(rouge pourpre, lésions profondes)

– Formation d'oedèmes:

Pas d'odème ...............................................................................................0
Très léger oedème.......................................................................................1
Léger oedème (contours bien définis, gonflement apparent)..........................2
Oedème moyen (épaisseur environ 1 mm) ....................................................3
Oedème grave (épaisseur supérieure à 1 mm
et surface supérieure à celle de la zone d'application) .....................................4

On additionne les chiffres obtenus pour l'érythème et pour l'oedème après vingt quatre heures et soixante douze heures, d'une part sur les six zones non scarifiées, d'autre part sur les six zones scarifiées.

On additionne les chiffres ainsi obtenus et on calcule la moyenne en divisant le total par 24.

Cette moyenne représente l'indice d'irritation primaire cutanée (I.P.).

Les résultats sont exprimés comme suit:

NON IRRITANT              $I.P. \leq 0,5$
LEGEREMENT IRRITANT   $0,5 \; I.P. \leq 2$
IRRITANT                  $2 \; I.P. \leq 5$
TRES IRRITANT         $5 \; I.P. \leq 8$

Les résultats obtenus pour chaque lapin figurent sur le Tableau IV ci-après.

L'indice ainsi déterminé est égal à 0,04 ce qui correspond à un produit qui est considéré légalement suivant l'Arrêté du 1er février 1982 paru au Journal Officiel du 21 février 1982, comme non irritant.

## TOXICSOTE A COURT TERME - DETERMINATION DE LA DL 50 L'appréciation de la dose léthale 50 consiste à déterminer la dose mortelle pour 50 des animaux mis en expérience : rats et O$^{\sigma}$ de souche Wistar.

Cette détermination suppose certaines contraintes à savoir:

– le volume utilisé doit être au maximum de 0,5 ml par 20 g de poids corporel;
– la concentration en principe actif doit être connue ainsi que la nature de l'excipient.

Compte tenu de la texture et de la composition de la crème d'algues GYFL le principe actif ne peut être identifié en tant que tel, nous avons donc opéré sur la crème d'algues entière sans adjonction d'excipient et choisi comme voie d'administration la voie orale par gavage gastrique.

Les doses choisies ont été échelonnées de 0,5 g à 2,5 g pour 100 g de poids corporel.

A la dose de 2,5 g pour 100 g de poids corporel, les morts que l'on a observés étaient en fait dûs à une mauvaise manipulation de l'opérateur (asphyxie par encombrement pulmonaire).

On en déduit donc que la DL 50 de crème d'algues GYFL est supérieure à 25 g/kg (facteur limitant: non possibilité d'absorption volumétrique).

## Tableau I

### Résultats d'analyses

Les résultats sont exprimés en pourcentage de produit humide

| Echantillons | Cendres | Substances Organiques | | |
|---|---|---|---|---|
| | | Protéines | Lipides | Glucides* |
| Gyfl | 0,312 % | 0,12 % | 0,02 % | 0,756 % |

\* exprimés en "équivalent glucose"

**Tableau II**

**Gyfl**

| | Méthode | Minéralisation | Précision % |
|---|---|---|---|
| Sodium | Spectrométrie de flamme (a) | 1 | ± 5 |
| Potassium | "        "      "    (a) | 1 | 4 |
| Lithium | "        "      " | 1 | 4 |
| Calcium | Absorption atomique (flamme) – AFNOR V18–108 | 1 | 4 |
| Magnesium | "        "        "        – (b) | 1 | 4 |
| Strontium | Spectrométrie de flamme | 1 | 4 |
| Chrome | Colorimétrie (Diphényl Carbazide) | 2 | / |
| Fer | Absorption atomique (flamme) – (c) | 2 | ± 5 |
| Manganèse | "        "        "        – (c) | 2 | 4 |
| Cuivre | "        "        (four)  – (c) | 2 | ±10 |
| Cobalt | "        "        " | 2 | / |
| Nickel | "        "        " | 2 | ±10 |
| Zinc | Absorption atomique (flamme) – (c) | 2 | ± 5 |
| Etain | "        "        (flour) | 2 | ±10 |
| Plomb | "        "        " | 2 | " |
| Cadmium | "        "        " | 2 | " |
| Molybdène | Clorimétrie (thiocyanate) | 2 | ± 5 |
| Antimoine | Clorimétrie (Rhodamine) | 2 | / |
| Titane | "        (acide chromotropique) | 2 | ±10 |
| Chlore | Volumétrie au nitrate d'argent – (a) | 3 | ± 3 |
| Phosphore | Colorimétrie à l'heptamolybdate | 4 | ± 5 |
| Azote | Kjeldalh (volumétrie) | 4 | ± 5 |
| Arsenic | Colorimétrie (cf AFNOR T 90201) | 2 | ±10 |
| Bore | Colorimétrie (Curcumin) AFNOR T 90201 | 3 | ± 3 |
| Iode | "        (méthode aux sels cériques) | 3 | ± 3 |
| Brome | "        (phénolsulfonephtaléine) | 3 | ± 5 |
| Soufre | Népholométrie (sous forme SO4) | 1 | ± 5 |
| Fluor | Colorimétrie (méthode au zirconium) AFNOR T 90004 | 3 | ± 5 |
| Mercure | Absorption atomique sous flamme AFNOR T 90113 | 5 | ±10 |
| Sélénium | Polarographie | 6 | ±10 |

**Observations:**

1: Calcination et reprise des cendres par HCl
2: Minéralisation humide H2SO4 + HNO3 + HClO4
3: Calcination en milieu alcalin – reprise des cendres par eau
4: Minéralisation humide (H2SO4 + H2O2)
5: Minéralisation humide (H2SO4 + HNO3 + KMNO)
6: Attaque à l'acide bromhydridque

**Légende:**

(a):     1e directive C.E.E. Aliments des animaux
(b):     4e directive C.E.E.
(c):     8e directive C.E.E.
(d):     3e directive C.E.E.

**Tableau III**

**Résultats de l'analyse**

|  |  |  | Gyfl |
|---|---|---|---|
| Sodium | (Na) | mg/kg | 816 |
| Potassium | (K) | " | 850 |
| Lithium | (Li) | µg/kg | ≤ 15 |
| Calcium | (Ca) | mg/kg | 150 |
| Magnesium | (Mg) | " | 118 |
| Strontium | (Sr) | " | 1,700 |
| Chrome | (Cr) | " | < 0,1 |
| Fer | (Fe) | " | 2,9 |
| Manganèse | (Mn) | " | 0,34 |
| Cuivre | (Cu) | " | 0,31 |
| Cobalt | (Co) | µg/kg | < 50 |
| Nickel | (Ni) | " | 50 |
| Molybdène | (Mo) | " | < 20 |
| Zinc | (Zn) | "2 | 2,0 |
| Etain | (Sn) | mg/kg | 17 |
| Plomb | (Pb) | " | < 0,1 |
| Cadmium | (Cd) | " | 0,042 |
| Antimoine | (Sb) | " | < 0,01 |
| Chlore | (Cl) | " | 973 |
| Phosphore | (P) | " | 176 |
| Azote | (N) | " | 188 |
| Arzenic | (As) | " | 3,0 |
| Bore | (B) | " | 1,41 |
| Iode | (I) | " | 1,8 |
| Brome | (Br) | " | 16 |
| Soufre | (S) | " | 222 |
| Titane | (Ti) | " | < 12 |
| Flour | (F) | " | 8,4 |
| Mercure | (Hg) | " | < 0,05 |
| Sélénium | (Se) | " |  |

**Tableau IV**

**Resultats individuels de determination de l'indice**

**D'irritation primaire de filtrat physiologique gyfl**

| | Résultats après 24 heures | | | | Résultats après 72 heures | | | |
| | Cote scarifié | | Cote non scarifié | | Cote scarifié | | Cote non scarifié | |
| Lapin | Oedème | Erythème | Oedème | Erythème | Oedème | Erythème | Oedème | Erythème |
|---|---|---|---|---|---|---|---|---|
| 1 | 0 | 0 | 0 | 0 | 0 | 0 | 0 | 0 |
| 2 | " | 1 | " | 0 | " | " | " | " |
| 3 | " | 0 | " | 0 | " | " | " | " |
| 4 | " | 0 | " | 0 | " | " | " | " |
| 5 | " | 0 | " | " | " | " | " | " |
| 6 | " | 0 | " | " | " | " | " | " |
| Total | 0 | 1 | 0 | 0 | 0 | 0 | 0 | 0 |

Total General : 1
Indice D'irritation Primaire : 1/24 = 0,04

**Exemple 2: Filtrat d'artichaut ("GYFAR")**

On opère comme décrit ci-dessus pour les algues, notamment comme décrit dans l'exemple 1. On obtient un filtrat brun limpide.

8

On trouvera dans le tableau V ci-après les résultats d'analyse du filtrat d'artichauts.

**Exemple 3 Filtrat d'algues** (Ascophylum)

On opère comme décrit dans l'exemple 1 ci-dessus, mais en utilisant une algue Ascophylum.

On trouvera dans le tableau VI ci-après les résultats d'analyse de ce filtrat "GYFA" et dans le tableau VII ci-après les résultats d'analyse des cendres de ce filtrat.

L'invention peut s'appliquer à toutes les plantes contenant des éléments utiles; on citera encore par exemple le Calendula, la prêle, etc...

ETUDE DE L'ASSIMILATION DE CERTAINS ELEMENTS EN FONCTION DE LA PRESENCE ET DE LA QUANTITE DE FILTRAT D'ALGUES GYFL INGERE.

**Protocole opératoire**

– 10 rats WISTAR $O^{\nearrow}$ utilisés en expérimentation
– 4 rats WISTAR $O^{\nearrow}$ utilisés comme témoin
Nourriture utilisée: aliment U.A.R. REF. A.04
Le poids corporel des animaux est indiqué dans le
Tableau VIII ci-après.

**Tableau V**

**Resultats de l'analyse**

|  |  |  | GYFAR (artichauts) |
|---|---|---|---|
| SODIUM | (Na) | mg/kg | 1950 |
| POTASSIUM | (K) | mg/kg | 4200 |
| LITHIUM | (Li) | µg/kg | 120 |
| CALCIUM | (Ca) | mg/kg | 1700 |
| MAGNESIUM | (Mg) | mg/kg | 118 |
| STRONTIUM | (Sr) | mg/kg | 11,6 |
| CHROME | (CR) | mg/kg | < 0,1 |
| FER | (Fe) | mg/kg | 0,85 |
| MANGANESE | (Mn) | mg/kg | 4,75 |
| CUIVRE | (Cu) | mg/kg | 2,6 |
| COBALT | (Co) | µg/kg | < 50 |
| NICKEL | (Ni) | µg/kg | < 50 |
| MOLYBDENE | (Mo) | µg/kg | < 20 |
| ZINC | (Zn) | µg/kg | 0,38 |
| ETAIN | (Sn) | mg/kg | 57 |
| PLOMB | (Pb) | mg/kg | < 0,1 |
| CADIUM | (Cd) | mg/kg | 0,042 |
| ANTIMOINE | (Sb) | mg/kg | ≤ 0,01 |
| CHLORE |  | mg/kg | 5820 |
| PHOSPHORE |  | mg/kg | 353 |
| AZOTE |  | mg/kg | 611 |
| ARSENIC |  | mg/kg | 0,07 |
| BORE |  | mg/kg | 0,6 |
| IODE |  | mg/kg | 104 |
| BROME |  | mg/kg | 40 |
| SOUFRE |  | mg/kg | 301 |

LIPIDES 0,19 %   PROTIDES 0,38 %   GLUCIDES 0,31 %
DL 50 : > 25 g/kg

**Tableau VI**

**Resultats D'analyses**

**Gyfa**

### Substances Organiques

| | | |
|---|---|---|
| Proteines | 0,030 % | |
| Lipides | 0,005 % | |
| Glucides | 0,032 % | |

### Mineraux

| | | | | | | | |
|---|---|---|---|---|---|---|---|
| Sodium | (Na) | mg/kg | 290 | Plomb | (Pb) | mg/kg | <0,1 |
| Potassium | (K) | " | 370 | Cadmium | (Cd) | " | 0,124 |
| Lithium | (Li) | µg/kg | ≤ 2,5 | Antimoine | (Sb) | " | ≤ 0,01 |
| Calcium | (Ca) | " | 42 | Chlore | (Cl) | " | 387 |
| Magnesium | (Mg) | " | 53 | Phosphore | (P) | " | 145 |
| Strontium | (Sr) | " | 0,25 | Azote | (N) | " | 49 |
| Chrome | (Cr) | " | <0,1 | Arzenic | (As) | " | 0,77 |
| Fer | (Fe) | " | 8 | Bore | (B) | " | 1,53 |
| Manganese | (Mn) | " | 0,35 | Iode | (I) | " | 23 |
| Cuivre | (Cu) | " | 1,85 | Brome | (B2) | " | <1 |
| Cobalt | (Co) | µg/kg | <50 | Soufre | (S) | " | 31 |
| Nickel | (Ni) | " | 110 | Titane | (Ti) | " | <1 |
| Molybdene | (Mo) | " | <20 | Flour | (F) | " | 8,2 |
| Zinc | (Zn) | " | 0,57 | Mercure | (Hg) | " | <0,05 |
| Etain | (Sn | mg/kg | 15 | Selenium | (Se) | " | |

### Amino Acides (en % sur la M.S.)

| | | | |
|---|---|---|---|
| Alanine | 0,209 | Arcinie | 0,128 |
| Glycine | 0,175 | Lysine | 0,172 |
| Valine | 0,189 | Cysteine | 0,078 |
| Leucine | 0,243 | Methionine | 0,018 |
| Isoleucine | 0,133 | Tyrosine | 0,060 |
| Glutamique Acide | 0,534 | Phenylalanine | 0,154 |
| Aspatrique Acide | 0,476 | Histidine | 0,075 |
| Theonine | 0,156 | Proline | 0,135 |
| Serine | 0,146 | Tryptophane | 0,022 |

**Tableau VII**

**Resultat D'analyse**

Les résultats sont exprimés en pourcentage de produit humide

| Echantillons | Cendres | Substances Organiques | | |
|---|---|---|---|---|
| | | Proteines | Lipides | Glucides* |
| Gyfa | 0,138 % | 0,030 % | 0,005 % | 0,032 % |

* exprimés en "équivalent glucose"

**Tableau VIII**

| Rats | Poids corporel |
|------|----------------|
| $N_1$ | 126 g |
| $N_2$ | 118 g |
| $V_1$ | 120 g |
| $V_2$ | 127 g |
| $R_1$ | 130 g |
| $R_2$ | 115 g |
| $Bl_1$ | 110 g |
| $Bl_2$ | 105 g |
| $B_1$ | 120 g |
| $B_2$ | 120 g |
| $T_1$ | 135 g |
| $T_2$ | 130 g |
| $T_3$ | 118 g |
| $T_4$ | 121 g |

A partir du 23 septembre et jusqu'au 5 octobre, $N_1$ et $N_2$ ont reçu par gavage gastrique 0,5 ml de filtrat GYFL par jour. Les 4 et 5 octobre, l'eau de boisson des rats $N_1$, $N_2$, $V_1$, $V_2$, $R_1$, $R_2$, $BL_1$, $BL_2$, $B_1$, $B_2$ a été remplacée par une solution aqueuse à 10 % de filtrat GYFL.

– On n'a observé aucune anomalie dans le comportement des rats $N_1$ et $N_2$.

– La solution à 10 % de filtrat n'a pas été répétée en tant que boisson, la quantité bue était tout à fait comparable à celle habituellement ingérée.

**Etude de l'assimilation du glucose**

Après ingestion, au temps T, par gavage gastrique de 0,5 ml d'une solution de glucose au sein de différents excipients, on suit toutes les 15 minutes l'évolution de la glycémie des rats.

– Solution utilisée:

Solution n°1  Glucose 50 g
Eau qsp 1000 ml

Solution n°2  Glucose 50 g
Filtrat GYFL 100 ml
Eau qsp 1000 ml

Solution n°3  Filtrat GYFL 100 ml/l
Glycérine 10 ml/L
Glucose 200 g/L
Fructose 75 g/L
B1 20 mg/L
C 5 g/L
Sodium 3 g/L
Acide citrique pH 3,2 qsp
Arôme citron 1,5 g/L
Eau gazéifiée 1000 ml qsp

Les rats

– $N_1$ et $N_2$ ont reçu la solution n°1
– $R_1$ et $R_2$ ont reçu la solution n°1
– $B_1$ et $B_2$ ont reçu la solution n°2
– $T_1$ et $T_2$ ont reçu la solution n°1
– $BL_1$ et $BL_2$ ont reçu la solution n°3

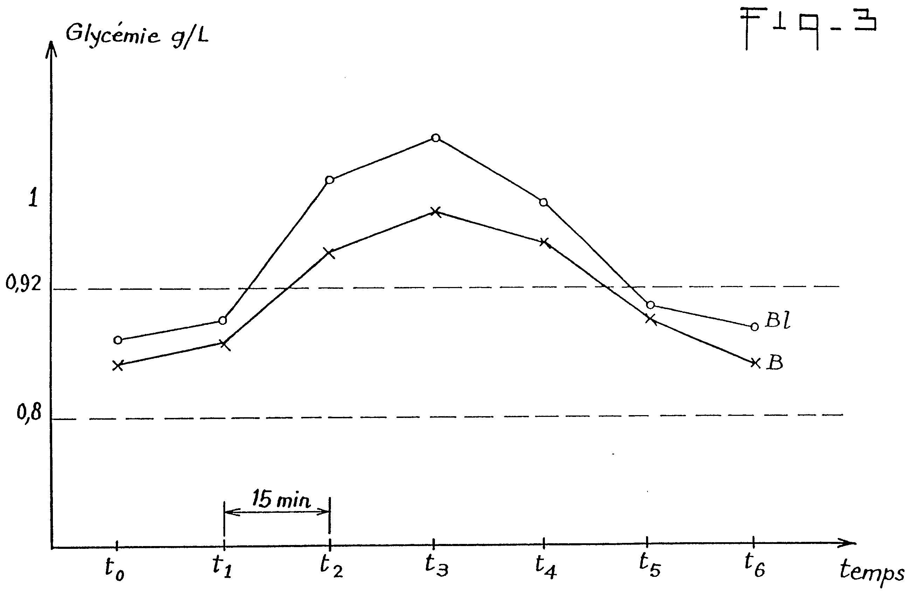

Les résultats sont rassemblés dans le Tableau IX ci-après et sur la Figure 3 annexée.

## Conclusions –

1. On observe un choc hypoglycémique net chez les rats non soumis auparavant à un régime d'algues et ayant ingéré une solution aqueuse de glucose exempte d'algues.
2. On observe un très léger choc hypoglycémique chez les rats soumis auparavant à un régime d'algues peu important et ayant ingéré une solution aqueuse de glucose.
3. On n'observe pas de choc hypoglycémique chez les rats soumis auparavant à un régime d'algues important ou inexistant, ayant ingéré une solution aqueuse de glucose à 10 % de filtrat GYFL. Il semble y avoir meilleure assimilation et utilisation du glucose ingéré.
4. Il semble y avoir stockage du glucose ingéré dans le cas des rats soumis auparavant à un régime d'algues important et de longue durée. En effet, il n'y a ni hyper ni hypoglycémie.

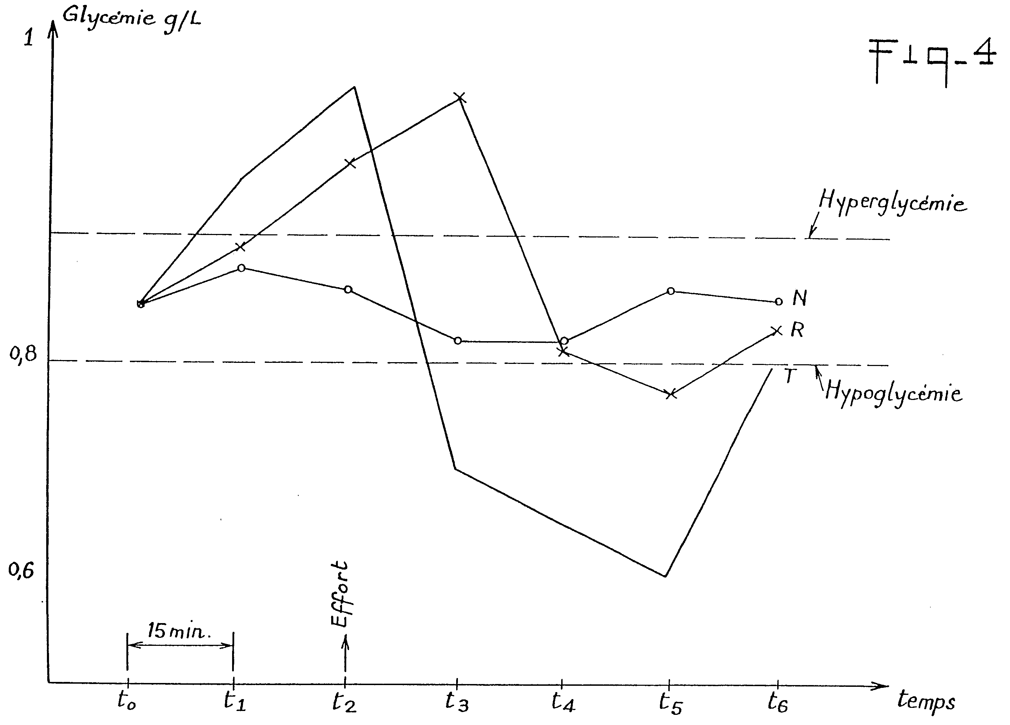

Pour conforter cette hypothèse, on a imposé un effort important au temps $T_2$ aux rats $R_2$ et $N_1$ : l'hypoglycémie du $T_2$ est importante, celle du $N_1$ est nulle, comme on peut le voir sur la Figure 4 annexée.

### Exemple 4

On prépare comme indiqué ci-dessus un filtrat d'algues Laminaria saccharina ou Ulva lactica.

On a constaté que, si l'on incorpore à des boissons l'extrait final ainsi obtenu, ou "filtrat physiologique", on obtient une augmentation tout à fait surprenante de la valeur énergétique de la boisson. Cette augmentation est sans rapport avec la valeur nutritive de l'apport d'algues, et est donc due à un effet de synergie.

La "crème d'algues" et le "protoexoplasma d'algues" ne peuvent pas être utilisés pour la réalisation de boissons.

Par contre, le "filtrat physiologique" défini ci-dessus peut être ajouté à toute boisson non alcoolinée, à raison en général de 2 à 10 %, notamment à des jus de fruits.

En fait, ce filtrat peut pratiquement constituer à lui seul une boisson. On peut ainsi concevoir une boisson constituée de 99 % de "filtrat physiologique" et de 1 % d'aromatisants et additifs naturels.

On trouvera dans le tableau X ci-après une liste préférée d'algues utilisables en alimentation.

### Tableau IX

### Resultats de glycemie

| | | $T_0$ | $T_1$ | $T_2$ | $T_3$ | $T_4$ | $T_5$ | $T_6$ | $T_n = T_{n-1} + 15'$ |
|---|---|---|---|---|---|---|---|---|---|
| Glucose + eau | $R_1$ | 0,86 | 0,90 | 0,98 | 1,05 | 0,80 | 0,75 | 0,83 | rats soumis à un régime d'algues peu |
| | $R_2$ | 0,87 | 0,92 | 0,99 | 1,03 | 0,83 | 0,78 | 0,84 | important et de courte durée |
| Glucose + eau + filtrat | $B_1$ | 0,88 | 0,89 | 0,93 | 0,99 | 0,95 | 0,89 | 0,86 | rats soumis à un régime d'algues peu |
| | $B_2$ | 0,83 | 0,83 | 0,96 | 0,98 | 0,99 | 0,92 | 0,83 | important et de courte durée |
| Boisson | $BL_1$ | 0,86 | 0,86 | 1,03 | 1,05 | 1,01 | 0,90 | 0,87 | rats soumis à un régime d'algues peu |
| | $BL_2$ | 0,87 | 0,90 | 1,01 | 1,06 | 1,00 | 0,89 | 0,88 | important et de courte durée |
| Glucose + eau | $T_1$ | 0,85 | 0,98 | 1,05 | 0,70 | 0,65 | 0,60 | 0,79 | rats non soumis à régime |
| | $T_2$ | 0,85 | 0,96 | 1,07 | – | 0,65 | 0,66 | 0,81 | d'algues |
| Glucose + eau | $N_1$ | 0,87 | 0,89 | 0,87 | – | 0,80 | 0,89 | 0,85 | rats soumis à un régime d'algues pendant |
| | $N_2$ | 0,88 | 0,88 | 0,86 | 0,82 | 0,87 | 0,86 | 0,89 | 12 jours auparavant (important et de longue durée) |

### Tableau X

### Liste des algues utilisables en alimentation

Genre

| | |
|---|---|
| Ulva | Enteromorpha |
| Laminaria | Cladophora |
| Rhodymenia | Chodium |
| Chondrus | Lomentaria |
| Fucus | Gastrocelonium |
| Spirulina | Ceramium |

**Tableau X (suite)**

| Bracchiomonas | Ectocarpus |
| Platymonia | Paonia |
| Ulothrix | Coradophylum |
| Alaria | Pelvetia |
| Himanthalia | Bifurcaria |
| Cystosera | Gelidium |
| Dilcea | Furcellaria |
| Cystoclonium | Gigartina |
| Callibepharis | Gracilaria |
| Paliara | Laurencia |

**Exemple 5**

On trouvera ci-dessous une variante moins préférée de l'invention, ne comportant pas l'étape finale d'ultrafiltration.

On a utilisé les algues Laminaria saccharina ou Ulva lactuca.

On prépare un protoexoplasma d'algues comme décrit ci-dessus (cf. figure 2).

On a constaté que, si l'on incorpore à des aliments l'extrait final ainsi obtenu, on obtient une augmentation tout à fait surprenante de la valeur énergétique des aliments. Cette augmentation est sans rapport avec la valeur nutritive de l'apport d'algues, et est donc due à un effet de synergie.

Cette propriété a été mise en évidence par l'essai suivant.

On a soumis un groupe de rats de souche WISTAR (lot témoin) à un régime composé d'aliment normalisé référence A.04 (composition dans le tableau XI ci-après), et deux autres lots de rats de même souche à un régime à base du même aliment complémenté par une "crème d'algue "de Laminaria saccharina (P2) pour l'un et par un "protoexoplasma" (P1) de la même variété d'algues pour l'autre.

Il est apparu dès le 10ème jour du traitement:

— une baisse significative du poids de la nourriture absorbée par jour et par rat,
— une courbe de poids, sensiblement équivalente, des rats traités par rapport à celle des témoins.

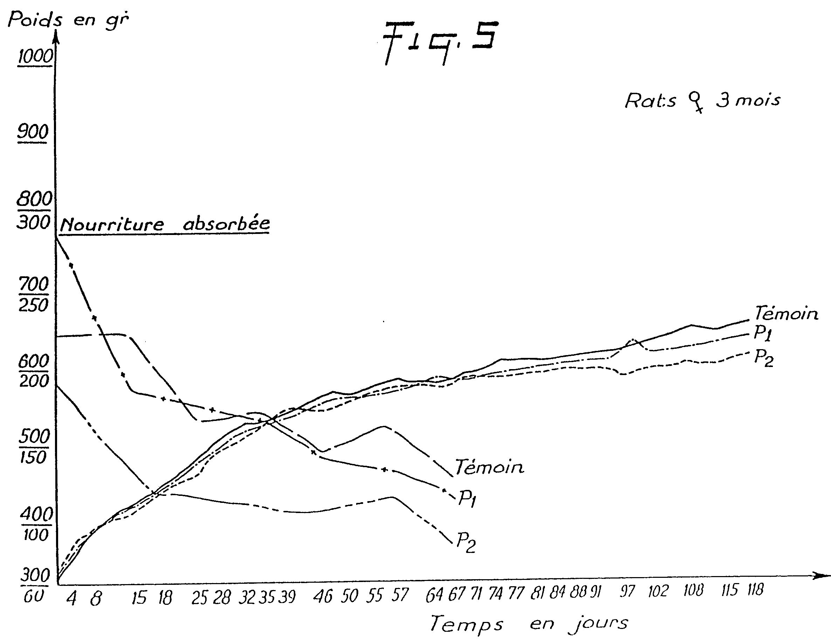

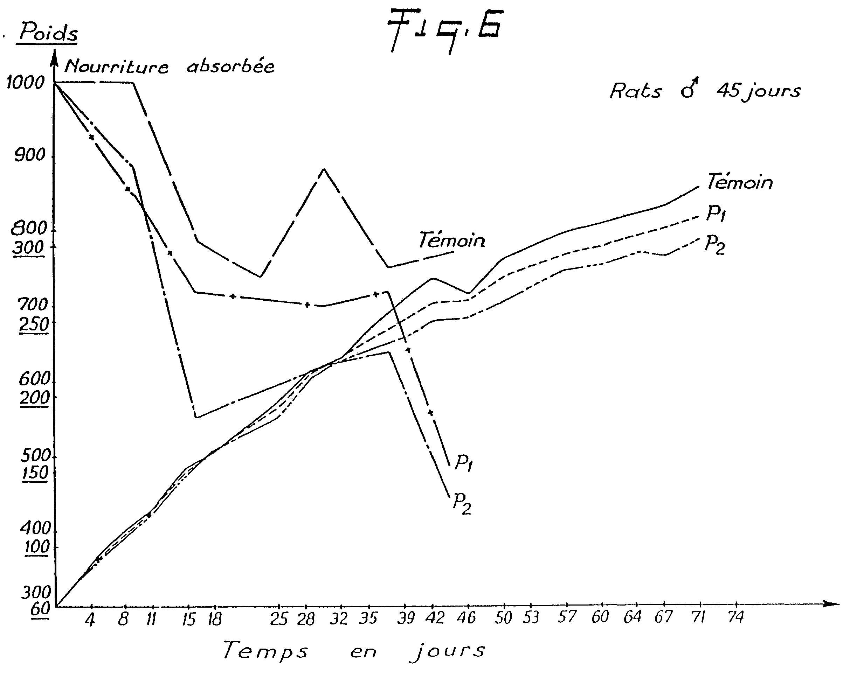

Les figures 5 et 6 du dessin annexé représentent ces courbes. De plus, bien que la baisse du poids de nourriture absorbée soit évidente, il n'apparaît pas d'autres phénomènes montrant une quelconque aggravation de l'état général des rats traités (chute de poids, état de prostration, état de stress...)

On peut donc déduire un caractère nutritionnel des produits d'algues testés difficilement compréhensible si on n'admet pas le caractère potentialisateur de ce type de produits. En effet, si on compare la composition de l'aliment U.A.R. A.04 (tableau XI ci-après) et celle des produits d'algues P1 et P2 (tableaux XII et XIII ci-après) on s'aperçoit qu'à poids égal les produits d'algues ont une valeur nutritionnelle nettement plus importante, par exemple au 30e jour de traitement 1 g de P1 correspond à 24, 14 g – 20 g = 4,86 g d'aliment UAR sans que leur composition centésimale soit comparable.

Pour conforter cette hypothèse, il a été pratiqué, le 67e jour de traitement, sur rats femelles, une évaluation de l'excrétion urinaire de 1-méthylhistidine des rats traités par rapport aux rats témoins par C.C.M sur plaque de silice et révélation par la ninhydrine, l'intensité de la couleur bleue observée étant fonction de la quantité d'acide amine ayant migré. L'évaluation de méthylhistidine excrétée par les rats traités par rapport aux rats témoins a été faite de visu.

Il n'a pas été observé d'augmentation significative de l'excrétion urinaire de 1-méthylhistidine.

Les algues considérées ne sont absolument pas toxiques.

On pourra donc utiliser les "crèmes d'algues" ou les "protoexoplasma d'algues" dans les aliments suivants, notamment

| Aliment | Crème d'algues | Protoexoplasma d'algues | % en poids de produit d'algue par rapport au poids de l'aliment |
|---|---|---|---|
| Pain | X | X | 0 à 20 |
| Produits panifiables | X | X | 0 à10 |
| Enrichissement des sauces | X | X | 0 à 80 |

| (suite) Aliment | Crème d'algues | Protoexoplasma d'algues | % en poids de produit d'algue par rapport au poids de l'aliment |
|---|---|---|---|
| Pâtés, conserves, plats cuisinés de viandes ou poissons | X | X | 0 à 20 |
| Pâtes alimentaires, crêpes, galettes | X | X | 0 à 25 |
| Confiserie | X | X | 0 à 20 |

Les essais suivants, non limitatifs, ont été réalisés.

**Exemple A**

On a fabriqué des crêpes selon une recette classique, en incorporant 5, 10 ou 15% de crème d'algue Laminaria saccharina.

**Exemple B**

On a fabriqué des bonbons en sucre cuit en incorporant dans une recette classique 1, 2, 3, 5 ou 10 % de crème d'algue Laminaria saccharina.

**Exemple C**

On a préparé un pâté de viande à 1, ou 2 %, de crème d'algue Laminaria saccharina, valeur en matières sèches.

**Exemple D**

On a préparé un gâteau de type "cake" contenant 1, ou 2 %, de crème d'algue Laminaria saccharina, valeur en matières sèches.

**Exemple E**

On a préparé un pain de mie à 1, ou 2 %, de crème d'algue Laminaria saccharina, valeur en matières sèches.

**Exemple F**

On a fabriqué une gomme à mâcher contenant 2 ou 5 % de crème d'algue Laminaria saccharina.

**Exemple G**

On a préparé à partir d'une pâte classique un pain contenant, par incorporation dans la pâte, 5 ou 10 % de crème d'algue Laminaria saccharina.

Pour aucune de ces préparations, il n'a été nécessaire de prendre de précautions spéciales. Il n'a été noté aucun goût ni odeur désagréable.

**Tableau XI**

**Aliment U.A.R A. 04**

Mineraux
(calculés en mg/kg)

| | Apport naturel (moyenne) | Apport par composé minéral | Totaux |
|---|---|---|---|
| P | 5 900 | 0 | 5 900 |
| Ca | 3 000 | 3 000 | 6 000 |
| K | 6 000 | 0 | 6 000 |
| Na | 800 | 1 600 | 2 400 |
| Mg | 2 000 | 130 | 2 130 |
| Mn | 40 | 40 | 80 |
| Fe | 90 | 150 | 240 |
| Cu | 14 | 15 | 29 |
| Zn | 40 | 45 | 85 |
| Co | 0,05 | 1,50 | 1,55 |

I apporté sous forme assimilable par algues marines

Vitamines
(calculées au kg)

| | | Apport naturel (moyenne) | | Apport synthétique | | Totaux | |
|---|---|---|---|---|---|---|---|
| Vitamine | A | 1 000 | UI | 7 500 | UI | 8 500 | UI |
| " | D 3 | 20 | " | 2 000 | " | 2 020 | " |
| " | B 1 | 6 | mg | 1 | mg | 7 | " |
| " | B 2 | 2 | " | 4,50 | " | 6,50 | " |
| " | B 3 | 10 | " | 6,50 | " | 16,50 | " |
| " | B 6 | 1,40 | " | 0,75 | " | 2,15 | " |
| " | B 12 | 0,009 | " | 0,01 | " | 0,19 | " |
| " | E | 18 | " | 15 | " | 33 | " |
| " | K 3 | 0,25 | " | 2,25 | " | 2,50 | " |
| " | PP | 60 | " | 15 | " | 75 | " |
| Ac. Folique | | 0,50 | " | 0 | " | 0,50 | " |
| Biotine | | 0,04 | " | 0 | " | 0,04 | " |
| Choline | | 1 180 | " | 400 | " | 1 580 | " |

**Tableau XI (suite)**

Formule %

| Céréales, sucre | 83,1 |
|---|---|
| Protéines végétales (tourteaux) | 6,9 |
| Protéines animales (poisson) | 6 |
| Composé Minéral Vitaminisé | 4 |

Analyse Moyenne %

| Valeur calorifique (Cal/kg) | 2 900 |
|---|---|
| Eau | 12 |
| Protides | 17 |
| Lipides | 3 |
| Glucides (E.N.A.) | 58,7 |
| Cellulose (Weende) | 4,3 |
| Minéraux | 5 |

Acides Amines
(calculés en mg/kg)

Arginine      9 800
Cystine      2 300
Lysine      8 500
Méthionine      3 200
Tryptophane      1 900
Glycine      8 100

Ration journalière du rat: suivant l'âge et le poids de 18 à 25 g.
Eau à volonté.

Ration journalière de la souris: suivant l'âge et le poids de 8 à 12 g.
Eau à volonté.

**Tableau XIX – composition chimique identifiee dans le**

**Protoexoplasma d'algues pheophycees ($P_1$)**

| Substances | En g pour 100 g de matière sèche | En mg/100 ml |
|---|---|---|
| Cendres totales ($H_2SO_4$) | 27,0 | |
| – Azote minéral | 0,63 | 37,9 |
| – Phosphore | 0,10 | 6,0 |
| – Iode (iodate, iodure) | 0,16 | 9,4 |
| – Chlorures | 6,84 | 410,7 |
| – Sodium | 5,23 | 314,0 |
| – Potassium | 6,47 | 388,0 |
| – Calcium | 0,85 | 51,0 |
| – Magnésium | 1,34 | 80,3 |
| – Fer | 0,19 | 11,7 |
| – Cuivre | 0,004 | 0,23 |
| – Zinc | 0,015 | 0,92 |
| – Manganèse | 0,005 | 0,31 |
| – Cobalt | – | < 0,007 |

Matières énergétiques

| – Azote aminé | 0,49 | |
|---|---|---|
| – Lipides totaux | 0,70 | |
| – Sucres identifiés: | | |
|     Mannitol | 6,1 | |
|     Fucose | 3,7 | |
|     Glucose | néant | |

*Vitamines*

| – Vitamines hydrosolubles: | | |
|---|---|---|
|     B1 (Thiamine) | | néant |
|     B2 (Riboflavine) | | 0,34 |
|     C (Acide ascorbique) | | néant |
| – Vitamines liposolubl: | | |
|     A (Rétinol) | | 1,5 |
|     Provit. A (ß carotène) | | 0,60 |
|     D (Calciférol) | | traces |
|     E (Tocophérol | | 4,86 |
|     K (Phylloquinone) | | 0,056 |

**Tableau XIII**

**Crème d'algues (P2)**
**(% en substances sèches)**

| | |
|---|---|
| Substances sèches | entre 12 et 15 |
| Azote minéral | 1,15 |
| Phosphore | 0,10 |
| Iode (iodate, iodure) | 0,10 |
| Chlorures | 3,54 |
| Sodium | 3,36 |
| Potassium | 4,60 |
| Calcium | 1,26 |
| Magnésium | 0,78 |
| Fer | 0,23 |
| Cuivre | 0,0015 |
| Zinc | 0,008 |
| Manganèse | 0,004 |

**Substances energetiques–**

| | |
|---|---|
| Azote aminé | 0,31 |
| Lipides totaux | 0,16 |
| Sucres identifiés | |
| – Mannitol | 1,48 |
| – Fucose | 0,90 |

**Vitamines**

| | | |
|---|---|---|
| –Hydrosolubles | | |
| B1 (thiamine) | 0,07 | mg/100 g MS |
| B2 (riboflavine) | 0,65 | mg/100 g MS |
| –Liposolubles | | |
| A (Retinol) | 0,39 | mg/100 g |
| Provit. A (ß carotène) | 13,47 | mg/100 mg |
| D (Calciférol) | traces | |
| E (tocophérol) | 1,17 mg/100 g MS | |
| K (phylloquinone) | 0,013 mg/100 g MS | |

**Phytohormones**

Gibberellin's (GA1, GA3, GA4, GA7, GA9) .......... 50 µg/100 g MS
Auxixines (iib, ANA, AIN, AIA)
Cytokinines – Abscissines

**Amino Acides**

| | |
|---|---|
| Alanine | 0,209 |
| Glycine | 0,175 |
| Valine | 0,189 |
| Leucine | 0,243 |
| Isoleucine | 0,133 |
| Acide glutamique | 0,534 |
| Acide aspartique | 0,476 |
| Thréonine | 0,156 |
| Sérine | 0,146 |
| Arginine | 0,128 |
| Lysine | 0,172 |
| Cystéine | 0,078 |
| Méthionine | 0,018 |
| Tyrosine | 0,060 |
| Phénylalanine | 0,154 |
| Histidine | 0,075 |
| Proline | 0,135 |
| Tryptophane | 0,022 |

## Revendications

1. Produit physiologique stérile résultant du traitement d'algues ou de plantes entières par un procédé du type comprenant une étape de cryobroyage consistant en une congélation des algues préalablement coupées, à -20/-50°C, suivie d'un broyage à cette température, dans un broyeur à couteaux ou à dents, puis d'un écrasement desdites algues broyées, dans un ou plusieurs broyeurs, à la température ambiante, afin d'obtenir une bouillie de dimension de particules de 100 à 50 ou 10μm, caractérisé en ce qu'il comprend, à la suite de cette première étape de cryobroyage, les étapes successives suivantes:

- broyage moléculaire dans des conditions choisies pour permettre l'éclatement cellulaire et la libération d'une grande quantité de liquide intracellulaire;
- décantation à grande vitesse;
- ultrafiltration, par exemple sur une membrane possédant un pouvoir de coupure inférieur à 20 000, en atmosphère stérile, de façon à récupérer ledit extrait sous forme d'un liquide stérile.

2. Nouveau produit selon la revendication 1, caractérisé en ce que l'étape d'ultrafiltration précitée est réalisée sur des filtres de carbone à couche d'oxyde de zirconium.

3. Nouveau produit selon la revendication 1 ou 2, caractérisé en ce que l'étape de broyage moléculaire précitée est réalisée dans un broyeur à piston, par exemple du type piston/clapet.

4. Nouveau produit selon la revendication 1, caractérisé en ce que le produit de départ est une algue.

5. Nouveau produit selon la revendication 1, caractérisé en ce que le produit de départ est une plante entière.

6. Nouveau produit selon la revendication 5, caractérisé en ce que le produit de départ est l'artichaut.

7. Procédé de préparation des nouveaux produits selon l'une quelconque des revendications 1 à 6, du type comprenant une étape de cryobroyage d'algues ou de plantes entières consistant en une congélation des algues, préalablement coupées, à -20/-50°C, suivie d'un broyage à cette température, dans un broyeur à couteaux ou à dents, suivie d'un écrasement desdites algues broyées, dans un ou plusieurs broyeurs, à la température ambiante, afin d'obtenir une bouillie de dimension de particules de 100 à 50 ou 10 μm, caractérisé en ce qu'il comprend à la suite de cette première étape de cryobroyage, les étapes successives suivantes:

- broyage moléculaire dans des conditions choisies pour permettre l'éclatement cellulaire et la libération d'une grande quantité de liquide intracellulaire;
- décantation à grande vitesse;
- ultrafiltration, par exemple sur une membrane possédant un pouvoir de coupure inférieur à 20 000, en atmosphère stérile, de façon à récupérer ledit extrait sous forme d'un liquide stérile.

8. Appareillage pour la mise en oeuvre du procédé selon la revendication 7, caractérisé en ce qu'il comporte la combinaison de broyeurs réfrigérés à -20/-50°C, de broyeurs à température ambiante, d'un broyeur moléculaire, d'une décanteuse à grande vitesse et d'un appareil d'ultrafiltration.

9. Utilisation des produits selon l'une quelconque des revendications 1 à 6 pour la préparation de médicaments, d'aliments ou de boissons, ou de compositions cosmétiques.

## Patentansprüche

1. Steriles physiologisches Produkt, das aus der Behandlung von Algen oder Vollpflanzen mittels eines Verfahrens resultiert, welches eine Kryozerkleinerungsstufe umfaßt, die im Einfrieren der zuvor geschnittenen Algen bei -20/-50°C mit anschließender Zerkleinerung bei dieser Temperatur in einer Schneid- oder Zahnscheibenmühle und dann Zermalmen der zerkleinerten Algen in einer oder mehreren Mühlen bei Umgebungstemperatur, besteht, um schließlich eine Brühe mit einer Teilchendimension von 100 bis 50 oder 10 μm zu erhalten, dadurch gekennzeichnet, daß es nach dieser ersten Kryozerkleinerungsstufe die folgenden aufeinanderfolgenden Stufen umfaßt:

- Molekularzerkleinerung unter Bedingungen, die derart gewählt sind, daß ein Zerplatzen der Zellen und die Freisetzung einer großen Menge an intrazellulärer Flüssigkeit möglich ist;
- Hochgeschwindigkeitsdekantierung;
- Ultrafiltration, beispielsweise über eine Membran mit einem Abtrennvermögen von weniger als 20 000 in steriler Atmosphäre zur Gewinnung des Extraktes in Form einer sterilen Flüssigkeit.

2. Neues Produkt nach Anspruch 1, dadurch gekennzeichnet, daß die Ultrafiltrationsstufe über Kohlenstoffilter mit einer Zirkonoxidschicht durchgeführt wird.

3. Neues Produkt nach Anspruch 1 oder 2, dadurch gekennzeichnet, daß die Stufe der Molarzerkleinerung in einer Kolbenmühle, beispielsweise des Typs mit Kolben/Klappe, durchgeführt wird.

4. Neues Produkt nach Anspruch 1, dadurch gekennzeichnet, daß das Ausgangsprodukt eine Alge ist.

5. Neues Produkt nach Anspruch 1, dadurch gekennzeichnet, daß das Ausgangsprodukt eine Vollpflanze ist.

6. Neues Produkt nach Anspruch 5, dadurch gekennzeichnet, daß das Ausgangsprodukt eine Artischocke ist.

7. Verfahren zur Herstellung von neuen Produkten nach einem der Ansprüche 1 bis 6, welches eine Stufe der Kryozerkleinerung von Algen oder Vollpflanzen umfaßt, die im Einfrieren der zuvor geschnittenen Algen bei -20/-50°C mit anschließender Zerkleinerung bei dieser Temperatur in einer Schneid- oder Zahnscheibenmühle und dann Zermalmen der zerkleinerten Algen in einer oder mehreren Mühlen bei Umgebungstemperatur, besteht, um schließlich eine Brühe mit einer Teilchendimension von 100 bis 50 oder 10 μm zu erhalten, dadurch gekennzeichnet, daß es nach dieser ersten Kryozerkleinerungsstufe die folgenden aufeinanderfolgenden Stufen umfaßt:

- Molekularzerkleinerung unter Bedingungen, die derart gewählt sind, daß ein Zerplatzen der Zellen und die Freisetzung einer großen Menge an intrazellulärer Flüssigkeit möglich ist;
- Hochgeschwindigkeitsdekantierung;
- Ultrafiltration, beispielsweise über eine Membran mit einem Abtrennvermögen von weniger als 20 000 in steriler Atmosphäre zur Gewinnung des Extraktes in Form einer sterilen Flüssigkeit.

8. Vorrichtung zur Durchführung des Verfahrens nach Anspruch 7, dadurch gekennzeichnet, daß sie Kühlmühlen mit -20/-50°C, Umgebungstemperaturmühlen, eine Molarmühle, einen Hochgeschwindigkeitsdekantierer und einen Ultrafiltrationsapparat in Kombination umfaßt.

9. Verwendung der Produkte nach einem der Ansprüche 1 bis 6 zur Herstellung von Medikamenten, Nahrungsmitteln oder Getränken oder kosmetischen Zusammensetzungen.

## Claims

1. Physiological product, resulting from the treatment of algae or whole plants with a process of the type comprising a cryocomminution step consisting in freezing the priorly cut algae, at -20/-50°C, followed by a milling at that temperature, in a mill with cutters or teeth, followed by a crushing of the milled algae, in one or more mills, at room temperature, in order to obtain a pulp of particle size from 100 to 50 or 10 μm, characterized in that it comprises, following said first cryocomminution step, the following steps:

- molecular milling in conditions selected so as to permit the molecular bursting and the release of a large quantity of intracellular liquid;
- high speed decantation;
- ultrafiltration, for example on a membrane having a cutting power less than 20 000 in sterile atmosphere, so as to recover said extract in sterile liquid form.

2. Novel product according to claim 1, characterized in that said ultrafiltration is conducted on charcoal filters with a layer of zirconium oxide.

3. Novel product according to claim 1 or 2, characterized in that said molecular milling is conducted in a mill incorporating a piston, for example of the piston/valve type.

4. Novel product according to claim 1, characterized in that the starting product is an alga.

5. Novel product according to claim 1, characterized in that the starting product is a whole plant.

6. Novel product according to claim 5, characterized in that the starting product is artichoke.

7. Process for preparing the novel products according to any one of claims 1 to 6, of the type comprising freezing the priorly cut algae, at -20/-50°C, followed by a milling at that temperature, in a mill with cutters or teeth, followed by a crushing of the milled algae, in one or more mills, at room temperature, in order to obtain a pulp of particle size from 100 to 50 or 10 μm, characterized in that it comprises, following said first cryocomminution step, the following steps:

- molecular milling in conditions selected so as to permit the molecular bursting and the release of a large quantity of intracellular liquid;

— high speed decantation;
— ultrafiltration, for example on a membrane having a cutting power less than 20 000 in sterile atmosphere, so as to recover said extract in sterile liquid form.

8. Apparatus for carrying out the process according to claim 7, characterized in that it comprises the combination of mills refrigerated to -20/-50°C, ambient temperature mills, a molecular mill, a turbo-decanter and an ultra-filtration apparatus.

9. Application of the products according to any one of claims 1 to 6, in the preparation of drugs, foods or drinks, or cosmetic compositions.

Fiq-1

Fiq-2

Fig-3

Fig-4

EP 0 160 650 B1

Fig. 5

EP 0 160 650 B1

Fig. 6